# FASCICULE DE BREVET EUROPEEN

(11) **EP 2 200 681 B1**
(45) Date de publication et mention de la délivrance du brevet: **11.06.2014**
(21) Numéro de dépôt: 08845593.6
(22) Date de dépôt: 22.10.2008
(51) Int. Cl.: A61M 5/32, A61M 5/31

(54) **DISPOSITIF DE SERINGUE COMPRENANT UN CORPS DE SERINGUE ET UN MANCHON DE SUPPORT**
SPRITZENVORRICHTUNG MIT EINEM SPRITZENKÖRPER UND EINER LAGERUNGSHÜLSE
SYRINGE DEVICE COMPRISING A SYRINGE BODY AND A BEARING SLEEVE

(30) Priorité: 23.10.2007 FR 0758497
(43) Date de publication de la demande: 30.06.2010
(73) Titulaire: Tech Group Europe Limited, Dublin 2 (IE)
(72) Inventeur: CHEVALLIER, Stéphane, F-77165 Saint-Soupplets (FR); CHEVALLIER, Jean-Michel, F-95880 Enghien-Les-Bains (FR)
(74) Mandataire: Lippert, Stachow & Partner
(86) Numéro de dépôt international: PCT/FR2008/051908
(87) Numéro de publication internationale: WO 2009/056735

(56) Documents cités:
- WO-A-03/068298
- FR-A- 2 861 598
- US-A1- 2007 239 117

## Description

La présente invention concerne un dispositif de seringue comprenant un corps de seringue ayant une direction axiale et un manchon de support à l'intérieur duquel ce corps est disposé, le manchon ayant au moins une patte de retenue coopérant avec le corps de seringue, à une extrémité proximale de ce dernier, pour retenir ledit corps par rapport au manchon.

Au sens de la présente invention, le terme « seringue » englobe aussi bien une seringue ayant une collerette à son extrémité proximale, qu'un objet du type d'une carpule ou d'une ampoule du type utilisé en particulier dans les domaines de la cosmétique ou de la pharmacie, pourvu ou non d'une telle collerette. Ainsi le « corps de seringue » au sens de l'invention présente une forme générale cylindrique et peut contenir en son intérieur un fluide liquide, gazeux ou pâteux, qui peut être expulsé par son extrémité distale, par exemple sous l'action d'un piston, disposé dans le corps et poussé vers cette extrémité distale.

Le manchon de support peut simplement servir à supporter le corps de seringue en son intérieur. Il peut également faire partie d'un dispositif de support de sécurité pour seringue, par exemple du type connu par EP 1 235 603 et par EP 1 474 194. D'autres corps de seringue sont connus par WO03/068298 et FR-A-2861598.

Dans ces dispositifs connus, le corps de seringue est maintenu à l'intérieur du manchon de support, en général sans pouvoir se déplacer axialement par rapport à lui. La ou les pattes de retenue du manchon de support permettent en effet une retenue efficace du corps de seringue par rapport au manchon, dans la direction axiale, grâce à leur coopération avec l'extrémité proximale du corps de seringue. Toutefois, dans les dispositifs connus, le corps de seringue peut tourner par rapport au manchon de support, selon une rotation autour de l'axe définissant la direction axiale.

Dans certains cas, cette liberté en rotation peut être tolérée car elle n'influence pas le fonctionnement du dispositif ni, en particulier, les opérations préparatoires à l'injection du produit contenu dans le corps de seringue. Dans d'autres cas, il est au contraire nécessaire de pouvoir caler le corps de seringue en rotation par rapport au manchon. En effet, il peut arriver que le corps de seringue soit tel qu'une aiguille d'injection ou un embout doive être rapporté à l'extrémité distale du corps de seringue par une rotation, en particulier par vissage. Il peut également arriver que le corps de seringue présente initialement une aiguille d'injection ou un embout, protégée par un capuchon de protection, lequel doit être ôté par un mouvement de dévissage, pour permettre une injection.

Il convient alors que le corps de seringue soit calé en rotation pour permettre soit la mise en place de l'aiguille, soit la séparation, par rapport au corps de seringue, du manchon de protection.

De manière générale, l'invention concerne un dispositif du type précité, et a pour but de permettre, de manière simple, un calage en rotation du corps de seringue par rapport au manchon de support et ce, quelles que soient les raisons pour lesquelles ce calage en rotation est nécessaire.

Ce but est atteint grâce au fait que ladite extrémité proximale du corps de seringue est solidaire avec une surface de calage qui présente des crénelures radiales formant des creux et des bosses, et la patte de retenue est apte à être insérée dans un creux pour empêcher une rotation relative du corps de seringue et du manchon de support perpendiculairement à la direction axiale.

Grâce à l'invention, la patte de retenue du manchon de protection sert non seulement à réaliser la retenue axiale évoquée en préambule, mais également à réaliser le calage en rotation. Ainsi, la fabrication de l'ensemble du dispositif de l'invention reste extrêmement simple, de même que son assemblage.

En outre, la patte de retenue est en général du type élastique, s'effaçant élastiquement pour permettre l'insertion du corps de seringue et de la surface de calage solidaire de ce corps dans le manchon de support, et revenir ensuite dans sa position de retenue. En conséquence, après l'assemblage du dispositif, la patte de retenue coopère avec l'extrémité proximale du corps de seringue et, si cette patte n'est pas immédiatement dans une coopération avec un creux de la surface de calage permettant d'empêcher la rotation relative du corps de seringue et du manchon, il suffit de réaliser une telle rotation relative volontairement jusqu'à ce que la patte se trouve dans un creux de la surface de calage et puisse donc se caler par rapport à cette dernière. En conséquence, l'assemblage est extrêmement simple et ne rend obligatoire aucun pré-réglage visant à placer en registre la patte de retenue et le relief de calage avant l'assemblage.

De plus, les creux et les bosses sont simples à fabriquer et permettent un positionnement de calage très aisé car, pour que le calage soit réalisé, il suffit que la au moins une patte de retenue soit insérée dans l'un des creux.

Selon une première application, le corps de seringue présente une collerette située à l'extrémité proximale dudit corps, la surface de calage étant disposée sur l'extrémité proximale de la collerette.

Dans ce cas, la surface de calage peut être directement formée sur la collerette du corps de seringue, en une seule pièce avec ledit corps.

Cette possibilité permet de réaliser le calage angulaire sans augmenter le nombre de pièces constituant le dispositif. Elle est en particulier utilisable lorsque le corps de seringue est réalisé en matière plastique, par moulage, ce qui bien entendu n'exclut pas l'utilisation d'autres matériaux, par exemple du verre.

Selon une alternative, le dispositif comprend une pièce de calage rapportée à l'extrémité proximale du corps de seringue de manière à former ladite surface de calage.

Dans ce cas, le corps de seringue utilisé dans le dispositif de l'invention peut être de type tout à fait classique, et il suffit de rapporter à son extrémité proximale la pièce de calage pour rendre ce corps capable de réaliser un calage angulaire. Selon cette alternative, le corps de seringue peut présenter une collerette du type précité, ou bien, au contraire, ne pas présenter de collerette.

Selon cette alternative, avantageusement, la surface de calage est formée sur une partie de collerette de la pièce de calage, en saillie radiale par rapport à la paroi cylindrique du corps de seringue. Lorsque le corps de seringue comporte une collerette, la partie de collerette de la pièce de calage peut être placée sur la collerette du corps de seringue. La partie de collerette de la pièce de calage peut être plus ou moins rigide, sa rigidité axiale étant assurée par la collerette du corps de seringue sur laquelle elle repose.

Dans ce cas, la partie de collerette de la pièce de calage peut être disposée contre la surface de la collerette formée à l'extrémité proximale du corps de seringue, sans augmenter notablement les dimensions de l'ensemble constitué par le corps de seringue et la pièce de calage, par rapport aux dimensions initiales du corps de seringue. Ceci permet de réaliser le dispositif de l'invention dans un encombrement minimisé.

Lorsque la seringue est du type d'une carpule ou d'une ampoule, sans collerette, la partie de collerette de la pièce de calage est préférentiellement choisie pour être rigide dans la direction axiale, de manière à éviter de se fléchir sous l'effet des efforts exercés sur elle par la ou les pattes de retenue.

Avantageusement, le dispositif comprend, en outre, un manchon de protection, le manchon de protection et le manchon de support étant aptes à coulisser l'un par rapport à l'autre entre une configuration d'attente dans laquelle une aiguille placée à l'extrémité distale du corps de seringue peut dépasser par rapport au manchon de protection, et une configuration de protection dans laquelle l'aiguille est apte à être entourée par le manchon de protection.

Dans ce cas, l'invention est adaptée à un dispositif de sécurité.

L'invention sera bien comprise et ses avantages apparaîtront mieux, à la lecture de la description détaillée qui suit, de modes de réalisation représentés à titre d'exemples non limitatifs. La description se réfère aux dessins annexés, sur lesquels :
- la figure 1 montre un dispositif conforme à l'invention selon un premier mode de réalisation, selon une vue en perspective coupée dans un plan axial diamétral ;
- la figure 1A illustre une variante pour la partie d'extrémité distale du corps de seringue ;
- la figure 2 est une vue en coupe axiale dans un plan perpendiculaire au plan de coupe de la figure 1 ;
- la figure 3 montre, en coupe axiale, un exemple de pièce de calage pouvant faire partie du dispositif de l'invention ;
- la figure 4 est une vue de dessus de la pièce de la figure 3 ;
- les figures 5 et 6 sont des vues respectivement analogues aux figures 3 et 4 pour une variante de réalisation de la pièce de calage ;
- la figure 7 montre, en perspective, une partie d'extrémité proximale d'un corps de seringue faisant partie d'un autre mode de réalisation du dispositif de l'invention ; et
- la figure 8 montre, en coupe axiale, une variante pour un dispositif selon l'invention.

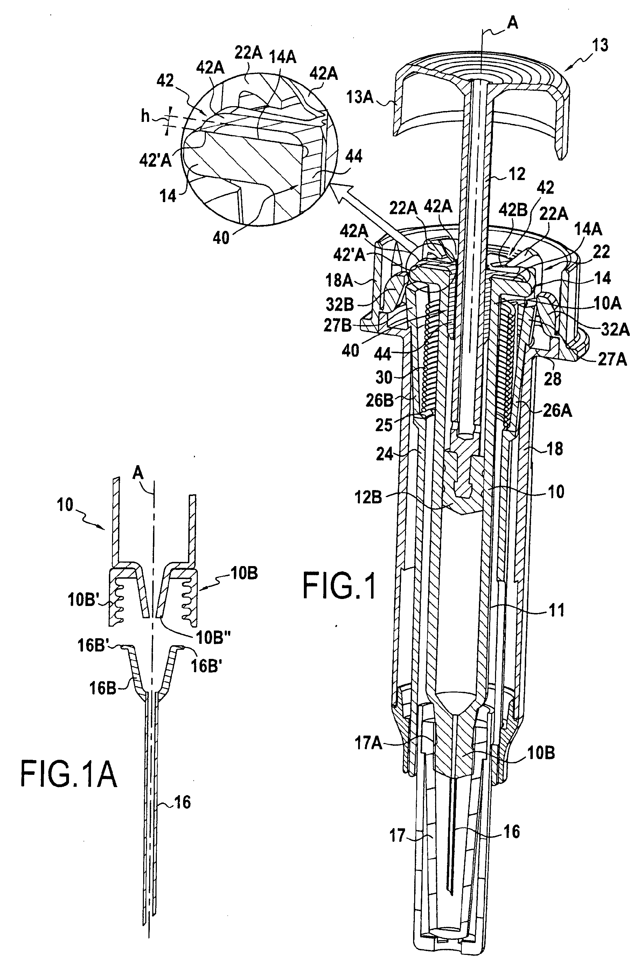

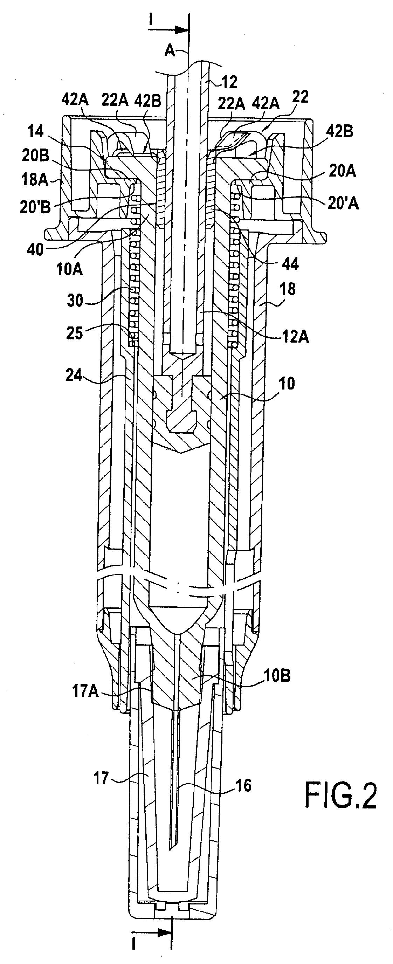

Le dispositif représenté sur les figures 1 et 2 comprend un corps de seringue 10 ayant une direction axiale selon l'axe A, et dans lequel un piston 12 peut coulisser selon cette direction axiale entre une position d'attente d'injection et une position de fin d'injection. Le piston est introduit dans le corps de seringue à partir de l'extrémité proximale 10A de ce dernier, cette extrémité proximale présentant une collerette 14 qui s'étend radialement. A son extrémité distale 10B, le corps de seringue porte une aiguille d'injection 16.

Au sens du présent texte, l'extrémité proximale est celle qui est la plus proche des doigts d'un utilisateur manipulant le dispositif pour réaliser une injection, tandis que l'extrémité distale est l'extrémité opposée, par laquelle le fluide contenu dans le corps de seringue est expulsé.

Le corps de seringue est supporté dans un manchon de support 18. A son extrémité proximale 18A, ce manchon de support 18 présente des épaulements de butée 20A et 20B (voir figure 2), ainsi que des pattes de retenue 22. On voit que la collerette 14 est retenue entre les épaulements 20A et 20B sur lesquels elle repose et les pattes de retenue 22. Le corps de seringue est donc calé axialement par rapport au manchon de support 18. En l'espèce, quatre pattes de retenue 22 sont prévues, mais leur nombre pourrait bien entendu être différent.

Le dispositif représenté sur les figures 1 et 2 comporte également un manchon de protection 24 qui est apte à coulisser par rapport au manchon de support 18 entre une position d'attente et une position de protection. Sur la figure 1, on voit que le manchon de protection 24 est retenu dans sa position d'attente par rapport au manchon de support 18 par des pattes longitudinales 26A et 26B dont les extrémités proximales, respectivement 27A et 27B sont conformées en crochets s'accrochant sur un épaulement annulaire 28 du manchon de support 18. Un ressort 30 est disposé entre la surface inférieure respectivement 20'A et 20'B des épaulements 20A et 20B et un épaulement annulaire interne 25 du manchon de protection. Le dispositif comporte encore des pattes de relais, respectivement 32A et 32B qui sont solidaires de l'extrémité proximale 18A du manchon de protection 18. La tête 13 du piston 12 porte une jupe axiale 13A.

On comprend que, en fin d'injection, cette jupe axiale 13A vient solliciter les pattes de relais 32A et 32B en flexion vers l'axe A, ce qui provoque également une flexion dans le même sens des pattes 26A et 26B afin que leurs extrémités proximales 27A et 27B conformées en crochets échappent à l'épaulement 28. Grâce à l'effort de rappel exercé par le ressort 30, le manchon de protection 24 peut alors se déplacer en coulissement vers l'extrémité distale par rapport au manchon de support 18, pour venir entourer l'aiguille et protéger ainsi cette dernière.

Pour ce qui vient d'être décrit, le dispositif représenté sur la figure 1 est conforme à celui que divulgue EP 1 474 194 et ne sera donc pas décrit plus en détail.

Ce n'est qu'un exemple de réalisation puisque, comme il a été indiqué précédemment, on pourrait prévoir que le dispositif comprenne, à titre de manchon, seulement un manchon de support, sans manchon de protection.

Par ailleurs, de manière connue, on pourrait prévoir un mode de retenue différent entre les manchons de protection et de support, permettant par exemple, en fin d'injection, une remontée de l'ensemble constitué par le corps de seringue et le manchon de support à l'intérieur du manchon de protection.

Ainsi, le dispositif de l'invention peut être utilisé dans tout type de système comprenant un corps de seringue retenu dans un manchon de support par sa collerette axiale et par des pattes de retenue que présente ce manchon.

On voit sur les figures 1 et 2 que l'aiguille 16 est entourée par un capuchon de protection 17 qui est retenu de manière amovible par rapport à l'extrémité distale 10B du corps de seringue. En l'espèce, le capuchon de protection est retenu par vissage sur la périphérie externe du corps de seringue, comme indiqué en 17A sur les figures 1 et 2. Son dévissage nécessite que le corps de seringue soit fixe en rotation par rapport au manchon de support, tenu par l'utilisateur.

Sur la figure 1A, l'extrémité distale 10B du corps de seringue 10 est apte à coopérer par vissage avec l'aiguille. L'extrémité 10B du corps de seringue présente en effet une bague de vissage 10B' qui entoure son embout 10B" avec un espace. Cette bague est filetée sur sa périphérie interne tournée vers l'axe A. L'aiguille 16 est solidaire, à son extrémité proximale, d'un raccord 16B qui est apte à être placé autour de l'embout 10B" en étant vissé, par ses ergots 16B', dans la bague 10B'. Pour pouvoir ainsi monter l'aiguille à l'extrémité du corps 10, une fois que celui-ci est en place dans le manchon de support, il faut que ce corps 10 soit fixe en rotation par rapport à ce manchon tenu par l'utilisateur.

Selon l'invention, l'extrémité proximale de la collerette 14 (plus précisément sa surface d'extrémité proximale 14A orientée transversalement à l'axe A) présente une surface de calage permettant un calage en rotation du corps de seringue par rapport au manchon de support. En l'espèce, selon le mode de réalisation des figures 1 et 2, cette surface de calage est formée grâce à une pièce de calage 40 qui est rapportée à l'extrémité proximale 10A du corps de seringue 10.

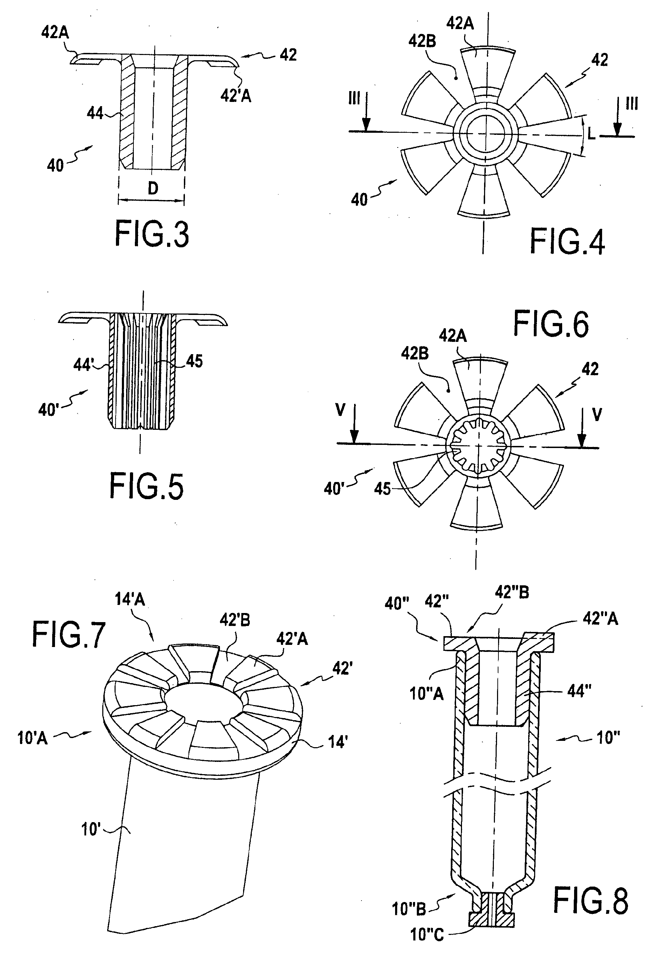

La pièce de calage 40 comprend une partie de collerette 42 (voir également figures 3 et 4) qui s'étend globalement radialement et qui est placée sur la collerette 14 du corps de seringue, plus précisément en étant disposée contre la surface d'extrémité proximale 14A. La partie de collerette 42 présente des ailettes 42A qui sont orientées sensiblement radialement et qui forment des bosses de la surface de calage, ces ailettes étant séparées les unes des autres par des encoches 42B qui forment des creux de la surface de calage. En l'espèce, les ailettes sont séparées par des découpes, les encoches étant traversantes dans la direction axiale. On pourrait bien entendu imaginer que ces encoches correspondent simplement à des zones de la collerette 42 dans lesquelles l'épaisseur de cette collerette, mesurée dans la direction axiale, serait inférieure à l'épaisseur qu'elle présente dans les zones des ailettes.

La pièce de calage 40 présente également une partie axiale tubulaire 44 qui, comme on le voit sur les figures 1 et 2, est insérée dans le corps de seringue 10, à partir de l'extrémité proximale 10A de ce dernier. La largeur L des encoches 42B, mesurée dans la direction circonférentielle de la collerette 42, correspond à la largeur des parties actives de retenue des pattes de retenue 22, mesurée également dans la direction circonférentielle autour de l'axe A. On voit en l'espèce que ces parties actives de retenue sont formées par des têtes d'accrochage 22A des pattes de retenue 22, dont la largeur va en diminuant en se rapprochant de l'axe A, c'est-à-dire en se rapprochant de leurs extrémités libres. Ces têtes d'accrochage sont formées par les extrémités des pattes, qui sont courbées vers l'axe A de manière à s'étendre sensiblement transversalement. De même, la largeur L des encoches 42B va en diminuant dans le même sens.

Les dimensions diamétrales externes D de la partie tubulaire 44 de la pièce de décalage correspondent aux dimensions diamétrales internes du corps de seringue 10, à l'extrémité proximale 10A de ce dernier, de manière à permettre un engagement à force de cette partie tubulaire 44 dans ce corps de seringue. Pour solidariser la pièce de calage 40 avec le corps de seringue, on choisira avantageusement de réaliser cette pièce 40 dans un matériau à fort coefficient de frottement, par exemple une résine de synthèse, un élastomère ou un mélange des deux, de sorte que l'engagement à force précité génère, sur les surfaces de la pièce de calage 40 et du corps de seringue 10 qui sont en contact, des forces de frottement élevées, s'opposant à une rotation relative entre le corps de seringue et la pièce de calage. Dans ce cas, la solidarisation peut être obtenue sans autre moyen particulier. On pourrait toutefois, si besoin, assurer cette solidarisation par un moyen complémentaire, tel qu'une soudure ou de la colle.

Sur la variante des figures 3 et 4, la partie tubulaire 44 est lisse. Les figures 5 et 6 montrent une pièce de calage 40', qui est analogue à la pièce 40 des figures 3 et 4 et présente en particulier la même partie de collerette 42, avec les ailettes 42A et les encoches 42B. La pièce 40' se distingue de la pièce 40 par sa partie tubulaire 44' qui, sur sa périphérie interne (tournée vers l'axe A), présente des cannelures axiales 45. Ces cannelures axiales confèrent à la partie tubulaire 44' une élasticité en direction radiale. Ainsi, lors de l'insertion de la partie tubulaire 44' à l'extrémité proximale 10A du corps de seringue 10, ladite partie tubulaire 40' peut être légèrement comprimée radialement en rapprochant les parties saillantes des cannelures 45 les unes des autres de manière à diminuer ses dimensions radiales et à favoriser ainsi son insertion dans le corps de seringue. Du fait de son élasticité naturelle, la partie tubulaire 44' a ensuite une tendance à une expansion en direction radiale, ce qui la bloque à l'intérieur du corps de seringue.

On voit que, dans les deux variantes, les extrémités libres 42'A des ailettes sont légèrement incurvées vers l'extrémité distale. Comme on le voit sur la partie agrandie de la figure 1, ceci permet de maintenir la partie plane des ailettes 42A à une légère distance h de la surface d'extrémité proximale 14A de la collerette 14 du corps de seringue. On augmente ainsi l'effet de bosse obtenu par la présence des ailettes.

On voit sur la partie droite de la figure 1 que la partie active de retenue 22A de l'une des pattes de retenue 22 est disposée dans une encoche 42B de telle sorte que cette patte de retenue est calée angulairement entre les deux ailettes délimitant entre elles cette encoche.

En revanche, sur la partie gauche de la figure 1, la partie active 22A de la patte de retenue 22 est disposée entre la collerette 14 et l'une des ailettes 42A. En effet, il n'est pas nécessaire de choisir que le nombre d'encoches et leurs écarts angulaires correspondent exactement au nombre de pattes de retenue et aux écarts angulaires des parties actives de retenue 22A de ces dernières. Du fait de la flexibilité des ailettes 42A et des pattes de retenue 22, il est parfaitement possible d'obtenir le calage angulaire souhaité dès lors que l'une des pattes de retenue est disposée dans une encoche, même si ce n'est pas le cas pour les autres pattes de retenue. En fait, il suffit qu'au moins l'un des éléments constitués par la patte de retenue 22 et la partie de collerette 42 soit flexible élastiquement pour permettre un positionnement de calage angulaire aisé entre la patte de retenue et une encoche de la collerette. Ceci permet également, si plusieurs pattes de retenue sont présentes, d'obtenir un calage angulaire efficace même si seule l'une des pattes de retenue est disposée dans une encoche de la collerette.

La partie tubulaire 44 ou 44' de la pièce de calage 40 ou 40' occasionne une légère surépaisseur à l'intérieur du corps de seringue. Les dimensions diamétrales de la tige de piston 12 sont choisies pour que cette tige puisse passer à l'intérieur de la pièce 40 ou 40' pour être raccordée au bouchon 12B déjà présent dans le corps avant la mise en place de la pièce 40 ou 40' à l'extrémité proximale du corps de seringue. Cette surépaisseur permet d'empêcher l'extraction du piston et du bouchon 12B par l'extrémité proximale du corps 10 pour préserver le produit contenu dans ce corps.

Sur la figure 7, on a représenté la partie d'extrémité proximale 10'A d'un corps de seringue 10' selon un deuxième mode de réalisation. On voit que, dans ce cas, la surface de calage 42' est formée directement sur la collerette 14' de ce corps de seringue. En effet, cette collerette présente, faisant corps avec elle, une pluralité de reliefs saillants 42'A entre lesquels sont délimités des creux 42'B. La conformation ainsi obtenue est analogue à celle qui est obtenue avec le mode de réalisation précédent, une fois que la pièce de calage est disposée à l'extrémité proximale 10A du corps de seringue 10. En effet, dans les deux cas, l'alternance de creux et de bosses forme des crénelures radiales aptes à permettre une retenue angulaire efficace d'au moins la patte de retenue 22.

Sur la figure 8, on a représenté un corps de seringue 10" du genre carpule ou ampoule, dépourvu de collerette à son extrémité proximale 10"A qui est tubulaire. A son extrémité distale 10"B, par laquelle le fluide contenu dans le corps pourra être expulsé, le corps comporte un embout 10"C. Des moyens complémentaires (par exemple un adaptateur d'injection) pourront être rapportés à cette extrémité distale, par exemple par clippage ou vissage.

La pièce de calage 40" qui est rapportée à l'extrémité proximale 10"A de manière à être solidaire du corps de seringue, présente une partie de collerette 42" en saillie radiale par rapport à la paroi cylindrique du corps 10". Les creux et les bosses de calage sont formés dans cette partie de collerette, comme dans la collerette 14' de la figure 7.

La partie gauche de la coupe passe par un creux 42"B, tandis que la partie droite passe par une bosse 42"A.

Même dans les creux, l'épaisseur de la partie de collerette est choisie pour conférer à cette dernière la rigidité suffisante pour supporter, sans déformation nuisible au calage, les efforts exercés par la ou les pattes de calage.

La partie axiale tubulaire 44" de la pièce de calage 40" peut être analogue aux parties axiales 44 ou 44' des pièces 40 et 40' décrites en référence aux figures 4 et 5.

## Revendications

1. Dispositif de seringue comprenant un corps de seringue (10 ; 10' ; 10") ayant une direction axiale (A), et un manchon de support (18) à l'intérieur duquel ce corps est disposé, le manchon ayant au moins une patte de retenue (22) coopérant avec le corps de seringue, à une extrémité proximale de ce dernier pour retenir ledit corps par rapport au manchon,
**caractérisé en ce que** ladite extrémité proximale (10A, 10'A, 10"A) du corps de seringue est solidaire avec une surface de calage (42 ; 42' ; 42") qui présente des crénelures radiales formant des creux (42B ; 42'B ; 42"B) et des bosses (42A ; 42'A ; 42"A) et **en ce que** la patte de retenue (22) est apte à être insérée dans un creux (42B ; 42'B ; 42"B) pour empêcher une rotation relative du corps de seringue (10 ; 10') et du manchon de support (18) perpendiculairement à la direction axiale.

2. Dispositif selon la revendication 1, **caractérisé en ce que** le corps de seringue (10, 10') présente une collerette (14, 14') située à l'extrémité proximale dudit corps, la surface de calage (42, 42') étant disposée sur l'extrémité proximale (14A, 14'A) de la collerette.

3. Dispositif selon la revendication 2, **caractérisé en ce que** la surface de calage (42') est directement formée sur la collerette (14') du corps de seringue (10'), en une seule pièce avec ledit corps.

4. Dispositif selon la revendication 1 ou 2, **caractérisé en ce qu'**il comprend une pièce de calage (40 ; 40' ; 40") rapportée à l'extrémité proximale (10A ; 10"A) du corps de seringue (10 ; 10") de manière à former ladite surface de calage.

5. Dispositif selon la revendication 4, **caractérisé en ce que** la surface de calage est formée sur une partie de collerette (42 ; 42") de la pièce de calage (40 ; 40' ; 40"), en saillie radiale par rapport à la paroi cylindrique (11) du corps de seringue (10 ; 10").

6. Dispositif la revendication 5, **caractérisé en ce que** la partie de collerette (42) de la pièce de calage (40 ; 40') présente des ailettes sensiblement radiales (42A) formant des bosses de la surface de calage, lesdites ailettes étant séparées les unes des autres par des encoches (42B) formant les creux de la surface de calage.

7. Dispositif selon l'une quelconque des revendications 4 à 6, **caractérisé en ce que** la pièce de calage (40 ; 40' ; 40") présente une partie axiale tubulaire (44 ; 44' ; 44") insérée dans le corps de seringue (10 ; 10"), à partir de l'extrémité proximale (10A ; 10"A) de ce dernier.

8. Dispositif selon la revendication 7, **caractérisé en ce que** ladite partie axiale tubulaire (44' ; 44") présente des cannelures (45).

9. Dispositif selon l'une quelconque des revendications 1 à 8, **caractérisé en ce qu'**il comprend, en outre, un manchon de protection (24), le manchon de protection et le manchon de support (18) étant aptes à coulisser l'un par rapport à l'autre entre une configuration d'attente dans laquelle une aiguille (16) placée à l'extrémité distale (10B) du corps de seringue (10 ; 10') peut dépasser par rapport au manchon de protection (24), et une configuration de protection dans laquelle l'aiguille (16) est apte à être entourée par le manchon de protection (24).

10. Dispositif selon la revendication 5 et l'une quelconque des revendications 1 à 9, **caractérisé en ce qu'**au moins l'un des éléments constitués par la patte de retenue (22) et la partie de collerette (42) est flexible élastiquement.

## Patentansprüche

1. Spritzenvorrichtung, umfassend einen Spritzenkörper (10; 10'; 10") mit einer axialen Richtung (A) und eine Lagerungshülse (18), in der der Körper angeordnet ist, wobei die Hülse mindestens eine Haltelasche (22) hat, die mit dem Spritzenkörper an dessen proximalem Ende zusammenwirkt, um den Körper relativ zur Hülse zu halten, **dadurch gekennzeichnet, dass** das proximale Ende (10A, 10'A, 10"A) des Spritzenkörpers mit einer Keilverbindungsfläche (42; 42'; 42"), die radiale Verzahnungen aufweist, die Vertiefungen (42B; 42'B; 42"B) und Höcker (42A; 42'A; 42"A) bilden, fest verbunden ist und dass die Haltelasche (22) in eine Vertiefung (42B; 42'B; 42"B) eingesetzt werden kann, um eine relative Drehung des Spritzenkörpers (10; 10') und der Lagerungshülse (18) senkrecht zur axialen Richtung zu verhindern.

2. Vorrichtung nach Anspruch 1, **dadurch gekennzeichnet, dass** der Spritzenkörper (10, 10') an dem proximalen Ende des Körpers eine Manschette (14; 14') aufweist, wobei die Keilverbindungsfläche (42, 42') an dem proximalen Ende (14A, 14'A) der Manschette angeordnet ist.

3. Vorrichtung nach Anspruch 2, **dadurch gekennzeichnet, dass** die Keilverbindungsfläche (42') direkt an der Manschette (14') des Spritzenkörpers (10') und einstückig mit dem Spritzenkörper gebildet ist.

4. Vorrichtung nach Anspruch 1 oder 2, **dadurch gekennzeichnet, dass** diese ein Keilverbindungsteil (40; 40'; 40") aufweist, das an dem proximalen Ende (10A; 10"A) des Spritzenkörpers (10; 10") derart angebracht ist, dass es die Keilverbindungsfläche bildet.

5. Vorrichtung nach Anspruch 4, **dadurch gekennzeichnet, dass** die Keilverbindungsfläche an einem Manschettenbereich (42; 42") des Keilverbindungsteils (40; 40'; 40") gebildet ist und bezüglich der zylindrischen Wand (11) des Spritzenkörpers (10; 10") radial vorspringt.

6. Vorrichtung nach Anspruch 5, **dadurch gekennzeichnet, dass** der Manschettenbereich (42) des Keilverbindungsteils (40; 40') im Wesentlichen radiale Flügel (42A) aufweist, die Nasen der Keilverbindungsfläche bilden, wobei die Flügel durch Kerben (42B) voneinander getrennt sind, die die Vertiefungen der Keilverbindungsfläche bilden.

7. Vorrichtung nach einem der Ansprüche 4 bis 6, **dadurch gekennzeichnet, dass** das Keilverbindungsteil (40; 40'; 40") einen rohrförmigen axialen Bereich (44; 44'; 44") aufweist, der von dem proximalen Ende (10A; 10"A) des Spritzenkörpers (10; 10") in denselben eingesetzt ist.

8. Vorrichtung nach Anspruch 7, **dadurch gekennzeichnet, dass** der rohrförmige axiale Bereich (44'; 44") Längsnuten (45) aufweist.

9. Vorrichtung nach einem der Ansprüche 1 bis 8, **dadurch gekennzeichnet, dass** diese ferner eine Schutzhülle (24) aufweist, wobei die Schutzhülle und die Lagerungshülle (18) zueinander verschiebbar sind zwischen einer Bereitschaftskonfiguration, in der eine an dem distalen Ende (10B) des Spritzenkörpers (10; 10') platzierte Nadel (16) bezüglich der Schutzhülle (24) hervortreten kann, und einer Schutzkonfiguration, in der die Nadel (16) von der Schutzhülle (24) umschlossen werden kann.

10. Vorrichtung nach Anspruch 5 und nach einem der Ansprüche 1 bis 9, **dadurch gekennzeichnet, dass** zumindest eines der Elemente, die durch die Haltelasche (22) und den Manschettenbereich (42) gebildet werden, elastisch flexibel ist.

## Claims

1. Syringe device comprising a syringe body (10; 10'; 10") having an axial direction (A) and a bearing sleeve (18) inside which the body is arranged, wherein the sleeve has at least one holding lug (22) co-operating with the syringe body, at a proximal end of the latter, in order to hold the body in relation to the sleeve,
**characterized in that** said proximal end (10A, 10'A, 10"A) of the syringe body is fixed to a wedging surface (42; 42'; 42") having radial crenulations forming dips (42B; 42'B; 42"B) and bumps (42A; 42'A; 42"B) and that the holding lug (22) can be inserted into a dip (42B; 42'B; 42"B) in order to prevent a relative rotation of the syringe body (10; 10') and the bearing sleeve (18) perpendicularly to the axial direction.

2. Device according to claim 1, **characterized in that** the syringe body (10, 10') presents a collar (14, 14') at the proximal end of said body, wherein the wedging surface (42, 42') is disposed on the proximal end (14A, 14'A) of the collar.

3. Device according to claim 2, **characterized in that** the wedging surface (42') is directly formed on the collar (14') of the syringe body (10') and in one piece with the body.

4. Device according to claim 1 or 2, **characterized in that** it comprises a wedging piece (40; 40'; 40") attached to the proximal end (10A; 10"A) of the syringe body (10; 10") in such a manner that it forms the said wedging surface.

5. Device according to claim 4, **characterized in that** the wedging surface is formed on a part of the collar (42; 42") of the wedging piece (40; 40'; 40"), projecting radially to the cylindrical wall (11) of the syringe body (10; 10").

6. Device according to claim 5, **characterized in that** the collar part (42) of the wedging piece (40; 40') has substantially radial lobes (42A) forming bosses of the wedging surface, wherein the lobes are separated from each other by notches (42B) forming the cavities of the wedging surface.

7. Device according to one of the claims 4 to 6, **characterized in that** the wedging piece (40; 40'; 40") presents a tubular axial portion (44; 44'; 44") inserted in the syringe body (10; 10") from the proximal end (10A; 10"A) of the latter.

8. Device according to claim 7, **characterized in that** the tubular axial portion (44'; 44") presents longitudinal grooves (45).

9. Device according to one of the claims 1 to 8, **characterized in that** it further comprises a protection sleeve (24), said protection sleeve and said bearing sleeve (18) being slidable relative to each other between an attendance configuration in which a needle (16) placed at the distal end (10B) of the syringe body (10; 10') can protrude relative to the protection sleeve (24), and a protecting configuration in which the needle (16) can be surrounded by the protecting sleeve (24).

10. Device according to claim 5 and any one of the claims 1 to 9, **characterized in that** at least one of the elements formed by the holding lug (22) and the collar part (42) is elastically flexible.
